# EUROPEAN PATENT SPECIFICATION

(11) **EP 2 136 785 B1**
(45) Date of publication and mention of the grant of the patent: **23.03.2011**
(21) Application number: 08736327.1
(22) Date of filing: 17.04.2008
(51) Int. Cl.: A61K 9/107, A61K 31/397, A61K 31/7048

(54) **PROCESS FOR DOSING SELF-EMULSIFYING DRUG DELIVERY SYSTEMS**
VERFAHREN ZUR DOSIERUNG VON SELBSTEMULGIERENDEN ARZNEIMITTELABGABESYSTEMEN
PROCÉDÉ PERMETTANT LE DOSAGE DE SYSTÈMES AUTO-ÉMULSIONNANTS D'ADMINISTRATION DE MÉDICAMENTS (SEEDS)

(30) Priority: 18.04.2007 EP 07106435; 23.07.2007 US 951267 P
(43) Date of publication of application: 30.12.2009
(73) Proprietor: Sandoz AG, 4056 Basel (CH)
(72) Inventor: SCHWARZ, Franz, Xaver, A-6300 Wörgl (AT)
(74) Representative: Altmann, Andreas
(86) International application number: PCT/EP2008/054665
(87) International publication number: WO 2008/128960

(56) References cited:
- WO-A-02/24165
- WO-A-02/39983
- WO-A-94/16969
- US-A1- 2002 156 124
- US-A1- 2003 021 752
- US-A1- 2006 104 997

## Description

### Field of the Invention

The present invention relates to self-dispersing drug delivery systems and a process for dosing self-dispersing drug delivery systems.

### Background of the Invention

Self-dispersing drug delivery system are generally known from the state of the art. Self-dispersing drug delivery system are useful for administering lipophilic drugs or drugs which are sensitive to hydrolysis.

The use of many pharmaceutically active compounds is severely limited due to low aqueous solubility. These compounds, often described as "lipophilic" or "hydrophobic", do not dissolve well in water, and may even form a separate physical phase in aqueous solutions. The low solubility in the aqueous environment of the gastrointestinal tract results in poor and inconsistent bioavailability and hampers the development of pharmaceutical products.

Considerations of cost, safety and patient compliance motivate the search for effective oral formulations of hydrophobic therapeutic compounds. Conventional oral formulations of hydrophobic pharmaceutical drugs, while allowing the administration of higher concentrations of the compounds in a unit dose, and satisfying the concerns of expense, safety and patient convenience present additional problems.

One approach to increase the bioavailability of an insoluble, hydrophobic drug is to administer the drug in the form of an oil-in-water emulsion. In such an emulsion, the drug is dissolved in an oil phase which is finely dispersed in an aqueous phase.

The drug in the resulting droplets of oil in water is absorbed more readily in the small intestine as compared to the drug in a non-emulsified oil.

A problem with emulsions is, however, that they are thermodynamically unstable and have poor long-term storage stability since they often tend to coalescence, creaming/sedimentation or phase separation. It is inter alia not possible to fill oil-in-water emulsions into gelatine capsules since the high water content of the emulsion is incompatible with the capsule shell and would dissolve it.

Consequently, only a limited amount of oil can be added to form a practically usable emulsion. The limited amount of oil in turn limits the amount of the hydrophobic drug that can be added into the formulation and, consequently, the potency or concentration of the resulting formulation.

To overcome the limitations of aqueous and emulsion dosage forms for the administration of hydrophobic drugs, some drugs have been formulated in vehicles that are not themselves emulsions, but which readily form an oil-in-water emulsion when gently mixed in water or aqueous media. These compositions are termed self-emulsifying drug delivery systems (SEDDS). Self-emulsifying drug delivery systems are non-aqueous mixtures of oils, other non-aqueous solvents and surfactants, ideally isotropic, which spontaneously form an emulsion upon introduction into an aqueous medium under conditions of gentle agitation similar to those encountered in the gastrointestinal tract (Pouton, CW, Adv. Drug Delivery Rev. 1997 25: 47-58). Because the self-emulsifying drug delivery systems contain no aqueous components, a high concentration of the hydrophobic drug may be incorporated into the vehicle.

For example, WO 2005/037250 discloses a self-emulsifying drug delivery system useful for the adminstration of a water-insoluble drug such as a 2,6 -di-alkyl-4-silyl-phenglic antioxidant to a patient. The self-emulsifying drug delivery system comprises a hydrophilic surfactant with hydrophilic-lipophilic balance (HLB) value greater than 10, a digestible oil comprised of medium chain fatty acids esters of propylene glycol, and a non-aqueous protic solvent. Optionally, a soluble chelating agent and antioxidant may be added to enhance the stability of the phenolic antioxidant drug.

US2006/0104997 relates to pharmaceutical compositions and methods for the mucosal and oral administration of monoterpenes and derivatives thereof. The compositions of this invention further comprise one or more surfactants and cosolvents and are in the form of self-emulsifying compositions. The compositions of the invention may further comprise water-insoluble therapeutic agents, vaccines and diagnostics. Such agents include but are not limited to taxanes, steroids, topoisomerase inhibitors such as etoposide and other water-insoluble or lipophilic drugs.

US2002/0156124 discloses pharmaceutical compositions suitable for oral administration comprising paclitaxel, a solvent, a surfactant, a substituted cellulosic polymer, and optionally but preferably a P-glycoprotein inhibitor. The composition may further comprise a diglyceride or mixture of diglyceride and monoglyceride. The composition generates a supersaturated paclitaxel microemulsion upon contact with water resulting in improved oral bioavailability of paclitaxel.

US2003/0021752 relates to pharmaceutical formulations for use in the administration of lipophilic medicaments via mucosal surfaces. In particular the invention provides pharmaceutical formulations for use in administration of a lipophilic medicament via a mucosal surface which upon hydration form an emulsion containing the lipophilic medicament which is capable of adhering to a mucosal surface and allowing controlled release of the medicament. The invention further provides pharmaceutical formulations which contain, as active ingredients, specific combinations of cannabinoids in pre-defined ratios.

Typically, a self-emulsifying drug delivery system containing a hydrophobic pharmaceutical drug is orally ingested, i.e. in form of the oily composition or encapsulated in gelantine capsules, by a patient, the resulting composition disperses in the gut to form a fine emulsion that does not separate into an aqueous phase and an oil phase.

Additionally, the dosage forms known from the state of the art often present the problem that it is difficult to adjust the amount of the self-dispersing drug delivery system and therefore the amount of active compound to the individual needs of the patient, e.g. age or body weight of the patient. Therefore, there exists a need for application forms for self-dispersing drug delivery systems which avoid these disadvantages.

### Summary of the Invention

The present invention relates to self-dispersing drug delivery systems and a process for dosing self-dispersing drug delivery systems.

The present invention provides a process for dosing a self-dispersing drug delivery system comprising an active compound, said process comprising the step of
(i) using a dosage dispenser to dose a defined amount of said self-dispersing drug delivery system comprising an active compound, and
(ii) adding the defined amount of the self-dispersing drug delivery system comprising an active compound obtained in step (i) to an aqueous solution to obtain an emulsion.
   According to a further embodiment, the process additionally comprises the step
(iii) agitating the mixture obtained from step (ii) comprising the aqueous solution and the sell-dispersing drug delivery system comprising an active compound.

The skilled person will understand that it is the emulsion obtained from steps (ii) or (iii) above which is to be administered to the patient as a drink and that said administration should take place upon formation of the emulsion or shortly thereafter in order to avoid coalescence, creaming or phase separation. In other words, the self-dispersing drug delivery system comprising an active compound is prepared for administration as an emulsion after addition to an aqueous solution.

Preferably, the self-dispersing drug delivery system is a self-emulsifying drug delivery system according to the present invention.

Preferably, the active compound is a compound which is sensitive to hydrolysis, in particular a lipophilic compound which is sensitive to hydrolysis.

According to another aspect, the present invention provides a process wherein the self-dispersing drug delivery system and/or the aqueous solution contains at least one compound selected from the group consisting of pharmaceutical excipients, diluents, sweeteners, flavouring agents, and colouring agents.

According to a further embodiment, the defined amount of the self-dispersing drug delivery system in step (i) is obtained in a single-stroke of the dosage dispenser.

According to the present invention, the dosage dispenser can have marks to determine the amount of self-dispersing drug delivery system dosed.

According to another aspect, the present invention provides a process for preparing a pharmaceutical composition comprising the steps of
(i) using a dosage dispenser to dose a defined amount of a sell-dispersing drug delivery system comprising an active compound;
(ii) adding the defined amount of the self-dispersing drug delivery system comprising an active compound obtained in step (i) to an aqueous solution to obtain an emulsion.

The skilled person will understand that it is the emulsion obtained from step (ii) above which is to be administered to the patient as a drink and that said administration should take place upon formation of the emulsion or shortly thereafter in order to avoid coalescence, creaming or phase separation. The self-dispersing drug delivery system comprising an active compound is thus prepared for administration as an emulsion after addition to an aqueous solution.

According to another aspect, the present invention provides the use of a dosage dispenser for dosing a self-dispersing drug delivery system comprising an active compound. Preferably, the active compound is a compound which is sensitive to hydrolysis, in particular a lipophilic compound which is sensitive to hydrolysis.

According to a further aspect, the present invention provides a dosage dispenser containing a self-dispersing drug delivery system comprising an active compound. Preferably the active compound is a compound which is sensitive to hydrolysis.

The present invention also provides a combination comprising
(a) a dosage dispenser containing a self-dispersing drug delivery system comprising an active compound and being capable of dosing a defined amount of a self-dispersing drug delivery system comprising an active compound, and
(b) a product leaflet containing instructions that a defined amount of said self-dispersing drug delivery system is to be dispensed into an aqueous solution to obtain an emulsion and that said obtained emulsion is to be administered as a drink to the patient.

The present invention furthermore provides a combination comprising
(A) a dosage dispenser containing a self-dispersing drug delivery system comprising an active compound and being capable of dosing a defined amount of a self-dispersing drug delivery system comprising an active compound, and
(B) a container with a potable amount of an aqueous solution, wherein the defined amount of said self-dispersing drug delivery system is to be dispensed to form an emulsion which can be administered as a drink.

The present invention also provides a combination as described above comprising a dosage dispenser containing a self-dispersing drug delivery system comprising an active compound and being capable of dosing a defined amount of a self-dispersing drug delivery system comprising an active compound, wherein the viscosity of the self-dispersing drug delivery system comprising an active compound is in the range of 500 mPa^{·}s to 80 000 mPa^{·}s.

The present invention also provides a method of treating a disease condition comprising administering to a patient in need of such treating a therapeutically effective amount of the pharmaceutical composition obtainable according to a process according to the present invention.

Furthermore, the present invention provides a pharmaceutical formulation comprising the pharmaceutical composition obtainable according to a process according to the present invention and at least one pharmaceutically acceptable excipient.

Preferably, the formulation is suitable for treating a disease condition in a mammal, in particular in man. The skilled person will understand that it is the pharmaceutical formulation obtained by a process according to the present invention which is to be administered to the patient as a drink and that said administration should take place upon formation of the pharmaceutical formulation or shortly thereafter in order to avoid coalescence, creaming or phase separation.

The present invention also provides the use of a therapeutically effective amount of the pharmaceutical composition obtainable according to a process according to the present invention and at least one pharmaceutically acceptable excipient for the preparation of a medicament for treating a disease condition, preferably wherein said medicament is to be administered to a patient in need thereof

### Description of the Invention

In general, the present invention relates to self-dispersing drug delivery systems and a process for dosing self-dispersing drug delivery systems.

The present invention provides a process for dosing a self-dispersing drug delivery system comprising an active compound, said process comprising the step of
(i) using a dosage dispenser to dose a defined amount of said self-dispersing drug delivery system comprising an active compound, and
(ii) adding the defined amount of the self-dispersing drug delivery system comprising an active compound obtained in step (i) to an aqueous solution to obtain an emulsion.

According to step (i) of the present invention, a defined amount of a self-dispersing drug delivery system comprising an active compound is dosed using a dosage dispenser.

The self-dispersing drug delivery system comprising an active compound is preferably stored in the dosage dispenser according to the present invention. According to the present invention, self-dispersing drug delivery systems are self-emulsifying or self-suspending drug delivery systems, more preferably self-emulsifying drug delivery systems.

Therefore, the present invention also provides a process for dosing a self-emulsifying drug delivery system comprising an active compound, said process comprising the step of
(i) using a dosage dispenser to dose a defined amount of said self-emulsifying drug delivery system comprising an active compound, and
(ii) adding the defined amount of the self-emulsifying drug delivery system comprising an active compound obtained in step (i) to an aqueous solution to obtain an emulsion.

The skilled person will understand that it is the emulsion obtained from step (ii) above which is to be administered to the patient as a drink and that said administration should take place upon formation of the emulsion or shortly thereafter in order to avoid coalescence, creaming or phase separation. The self-dispersing drug delivery system comprising an active compound is thus prepared for administration as an emulsion after addition to an aqueous solution.

The self-dispersing drug delivery system comprising the active compound generally is a fluid, preferably a liquid under standard conditions. The process according to the present invention allows to dose the self-dispersing drug delivery system and therefore the active compound for any suitable application. The use of a dosage dispenser allows to dose defined amounts of the self-dispersing drug delivery system depending on the application and a patients needs without using any further device.

The present invention therefore has the advantage, that the amount of active compound can be easily adjusted to the individual application, e.g. the age and weight of the patient.

According to the present invention, a "defined amount" is any previously defined amount of the self-dispersing drug delivery system comprising the active compound which depends in each case amongst others on the application, the active compound, and the amount of the active compound in the self-dispersing drug delivery system.

A suitable amount of the self-dispersing drug delivery system comprising the active compound depend in particular on the concentration of the active compound in the self-dispersing drug delivery system and the amount of the active compound needed for a given application. Suitable amounts range depending on the dosage dispenser used and the self-dispersing drug delivery system comprising the active compound to be dosed in the range between 0.05 mg and 1.0 mg, in particular 0.1 mg and 0.9 mg, preferably 0.15 mg and 0.8 mg, for example 0.2 mg and 0.7 mg.

According to the present invention, any suitable dosage dispenser can be used for the process of the present invention as long as it is suitable for pharmaceutical applications. In the context of the present invention, a dosage dispenser is any device that is suitable for dispensing a liquid, comprising a container and a pumping device. The pumping device generally has an opening extending into the volume of the container which allows to pump the liquid out of the container.

The dosage dispenser suitable according to the present invention must fulfill the requirements for uniformity of dosage units of the US Pharmacopoeia 2008.

To ensure the consistency of dosage units, each unit in a batch should have a drug substance content within a narrow range around the label claim. Dosage units are defined as dosage forms containing a single dose or a part of a dose of drug substance in each unit. The term "uniformity of dosage unit" is defined as the degree of uniformity in the amount of the drug substance among dosage units. The uniformity of dosage units can be for example demonstrated by the content uniformity. Details regarding the determination of the content uniformity can be found for example in section 905 of the US Pharmacopoeia 2008.

In particular, the dosage dispenser preferably administers uniform dosage units with a content uniformity of F between 0.900 and 1.100, preferably between 0.970 and 1.030.

The dosage dispenser according to the present invention is suitable for dosing a defined amount. According to the present invention, the dosage dispenser also has means which allow to dose a defined amount of the liquid. The dosage dispenser can for example have marks, preferably marks on the container which allow to dose a defined amount of liquid. Suitable dosage dispensers have been described in the prior art and may be selected from those described, for example, in WO 01/78903 or WO 98/37978.

The use of a dosage dispenser according to the present invention has the advantage that the active compound comprised in the self-dispersing drug delivery system can be dosed with high accuracy and consistency. Furthermore, the amount of the self-dispersing drug delivery system comprising the active compound can be easily adjusted to a given application and the needs of the patient, in particular to the bodyweight of the patient. This makes the process of the present invention in particular advantageous for paediatric applications.

According to the present invention, any suitable self-dispersing drug delivery system comprising an active compound can be used. Preferably, any self-emulsifying drug delivery system comprising an active compound can be used according to the present invention. In the context of the present invention, one principal characteristic of self-emulsifying drug delivery systems is their ability to form fine emulsions, preferably oil-in-water emulsions or microemulsions upon contact with an aqueous phases. In the context of the present invention, one principal characteristic of self-suspending drug delivery systems is their ability to form fine suspensions upon contact with an aqueous phases.

The viscosity of the self-dispersing drug delivery system comprising an active compound can vary in wide ranges depending on the dosage dispenser used. Preferably it is in the range of 500 mPa^{·}s to 80 000 mPa^{·}s.

For the purpose of the present invention viscosity values are determined at 22°C with a Brookfield digital viscosimeter model DV-II according to the manual No. M/85-160-G after the apparatus has been calibrated against the supplier's calibration standard. The skilled person will learn to choose the appropriate spindle based on the instructions in the above-mentioned manual and depending on the viscisity of the sample to be measured.

Generally, upon contact with an aqueous phase, the composition completely forms a fine dispersion of mean particle size of less than about 250 µm, preferably less than about 150 µm, with a preferred range being less than about 50 µm, when measured by photon correlation spectroscopy (PCS).

Preferably, upon contact with an aqueous phase, the self-emulsifying completely forms a fine emulsion of mean particle size of less than about 50 nm, preferably less than about 30 nm, with a preferred range being from about 15 nm to 30 nm, when measured by photon correlation spectroscopy (PCS).

For example, a self-dispersing drug delivery system can instantly or spontaneously form fine dispersion, in particular a fine suspension or emulsions, on exposure to water or aqueous solutions without the need for specialized equipment. Generally, they do not require a hydrophilic cosolvent, and preferably are shelf-stable.

The microemulsion formed in contact with an aqueous phase preferably consists of substantially uniform and spherical droplets dispersed in a continuous medium. It is preferably substantially nonopaque, i. e., is transparent or opalescent.

While no agitation and/or emulsification equipment is required to obtain emulsification, agitation and/or emulsification equipment can be utilized.

The suspension formed in contact with an aqueous phase preferably consists of substantially uniform and spherical particles dispersed in a continuous medium. It is preferably substantially nonopaque, i. e., is transparent or opalescent.

While no agitation and/or suspension equipment is required to obtain suspension, agitation and/or suspension equipment can be utilized.

The self-dispersing drug delivery system can have any suitable composition known to the person skilled in the art according to the present invention.

Preferably, the compounds used for the preparation of the self-emulsifying drug delivery system and the composition have a sufficiently pleasant taste so as to leading to the formation of a palatable emulsion upon dosage into the aqueous solution.

A pleasant taste is advantageous, in particular for paediatric applications. Preferably the excipients provide a relatively pleasant taste, that is they have a bitterness value of below 5000, more preferably below 1000, even more preferably below 300 and most preferably below 100. Preferably also the active pharmaceutical ingredient(s) provide(s) a relatively pleasant taste, that is it (they) has (have) a bitterness value of below 5000, more preferably below 1000, even more preferably below 300 and most preferably below 100. The bitterness value is determined according to the procedure described in item 2.8.15 in the European Pharmacopoeia 6.0. In that manner the self-dispersing drug delivery system of the invention has the additional advantage of providing a drink upon emulsification in an aqueous solution which is pleasant for oral application.

In accordance with the invention, a self-emulsifying drug delivery system preferably includes a digestible oil; a pharmaceutically acceptable hydrophilic surfactant being capable of dispersing the oil into water or aqueous solution; a pharmaceutically acceptable non-aqueous protic solvent capable of forming an isotropic mixture with the oil and surfactant; optionally a pharmaceutically acceptable chelating agent soluble in a non-aqueous system; and optionally further pharmaceutically acceptable compounds soluble in a non-aqueous system.

Any effective surfactant, or effective combinations thereof may be used in accordance with the present invention. Acceptable surfactants for use in the self-emulsifying drug delivery systems include pharmaceutically acceptable surfactants that produce a substantial fraction or majority of droplets that are less than 50 nm in diameter, or more preferably less than 30 nm in diameter, when the self-emulsifying drug delivery systems form an emulsion.

The surfactants may be used in a self-emulsifying drug delivery system in any effective concentration, including, for example, in a concentration range of 5% to 80% (w/v).

Acceptable oil components include propylene glycol esters of medium chain fatty acids, such as propylene glycol dicaprylate/dicaprate, propylene glycol dipelargonate, and propylene glycol dilaurate. The oil components may be used in the self-emulsifying drug delivery system in any effective concentration, including, for example, in a concentration range of 5% to 80% (w/v). Preferably, the oils and surfactants used fort he preparation of the self-emulsifying drug delivery system have a sufficiently pleasant taste so as to leading to the formation of a palatable emulsion upon dosage into the aqueous solution as discussed above.

Suitable oils and surfactants with respect to the taste are in particular Peceol® (Glyceryloleat), Lauroglykol® FCC (Propylenglykolnonolaurat), Labrafac® lipo (Capryl/Caprintrigylceride), Span® 80 (Sorbitanoleat), Captex® 355 (Capryl/Caprintriglyceride), Propylenglykol, Isopropylmyristat or Labrafil®, Tween® 80 (Polysorbat), Ethyloleat (Ethyl-9-octadecenoat), Cremophor® RH 40, Plurol Oleique® (Polyglycerol-6-dioleat), or Miglyol. Particularly preferred are Labrafil®, Tween® 80 (Polysorbat), Ethyloleat (Ethyl-9-octadecenoat), Cremophor® RH 40, Plurol Oleique® (Polyglycerol-6-dioleat), or Miglyol.

Any effective non-aqueous protic solvent, or effective combinations thereof may be used. Acceptable non-aqueous protic solvents include any pharmaceutically acceptable aliphatic and aromatic solvent, or effective combinations thereof Examples of non-aqueous protic solvents include ethanol, benzyl alcohol, propylene glycol, polyethylene glycols and glycerol. The protic solvents may be used in the self-emulsifying drug delivery system in any effective concentration, including, for example, in a concentration range of 5% to 50% (w/v). Preferably, also the solvents used for the preparation of the self-emulsifying drug delivery system and the composition have a sufficiently pleasant taste so as to leading to the formation of a palatable emulsion upon dosage into the aqueous solution as discussed above.

Suitable optional chelating agents include any pharmaceutically acceptable chelating agent, such as citric acid, maleic acid, succinic acid, tartaric acid, EGTA (ethylene glycol-bis((3-aminoethyl ether) tetraacetic acid) and EDTA (ethylene diamine tetraacetic acid). Such chelating agents are commercially available in various forms, e. g., as sodium or potassium salts or as the free acids. Such chelating agents may be used in the self-emulsifying drug delivery system in any effective concentration, including, for example, in a concentration range of between 0.01% and 10% (w/v).

Any effective soluble antioxidant may optionally be used with the compositions of the present invention. Effective soluble antioxidants are pharmaceutically acceptable antioxidants that generally enhance the stability of an active compound in a self-emulsifying drug delivery system, while not detrimentally affecting the self-emulsifying drug delivery system itself Suitable optional soluble antioxidants include alphatocopherol, tocopherol acetate, vitamin E, polyethylene glycol succinate, propyl gallate, butylated hydroxytoluene and butylated hydroxanisole.

Soluble antioxidants may be used in the self-emulsifying drug delivery system in any effective concentration, including, for example, in a concentration range of between 0.01 % and 10% (w/v).

The self-dispersing drug delivery systems may be prepared in any effective manner. In one embodiment, the self-emulsifying drug delivery systems of the invention may be prepared by mixing the hydrophilic surfactant and the digestible oil component, followed by adding the non-aqueous protic solvent to form a clear isotropic mixture. The optional chelating agent may be added as a solution in the protic solvent, and the optional antioxidant is then added to the solution mixture. The active compound can be added to the self-emulsifying drug delivery system at any stage of the preparation of the self-emulsifying drug delivery system.

According to step (ii), the self-dispersing drug delivery system is added to an aqueous solution to form an emulsion according to the present invention. Preferably, an emulsion is at least partially formed.

The emulsion can then be used as means to apply the active compound, i.e. as the pharmaceutical composition.

In the context of the present invention, an aqueous solution is a solution which comprises water but can also comprise other solvents, in particular solvents which are miscible with water. The aqueous solution can also comprise other compounds such as salts or buffers, preferably compounds which are frequently used in pharmaceutical compositions, such as pharmaceutical acceptable salts. Tap water, for example, is a suitable aqueous solution according to the present invention, likewise juices, like orange or apple juice, may be used.

Preferably, the self-dispersing drug delivery system according to the present invention is a self-emulsifying drug delivery system.

Therefore, the present invention provides a process for dosing a self-dispersing drug delivery system comprising an active compound as described above, wherein the self-dispersing drug delivery system is a self-emulsifying drug delivery system.

For forming an emulsion, it is generally only necessary to bring the self-emulsifying drug delivery system into contact with an aqueous solution. However, it is also possible in the context of the present invention, that the formation of the emulsion is enhanced by any suitable measure, for example by agitating the mixture of the self-emulsifying drug delivery system and the aqueous solution.

The skilled person will understand that it is in each case the emulsion obtained which is to be administered to the patient as a drink and that said administration should take place upon formation of the emulsion or shortly thereafter in order to avoid coalescence, creaming or phase separation. The self-dispersing drug delivery system comprising an active compound is thus prepared for administration as an emulsion after addition to an aqueous solution.

Likewise, if the self-dispersing drug delivery system is a self-suspending drug delivery system, for the process for forming a suspension, it is generally only necessary to bring the self-suspending drug delivery system into contact with an aqueous solution. However, it is also possible in the context of the present invention, that the formation of the suspension is enhanced by any suitable measure, for example by agitating the mixture of the self-suspending drug delivery system and the aqueous solution.

Therefore, the present invention provides a process for dosing a self-dispersing drug delivery system comprising an active compound as described above, wherein the process additionally comprises the step
(iii) agitating the mixture obtained from step (ii) comprising the aqueous solution and the self-dispersing drug delivery system comprising an active compound.

The skilled person will understand that it is the emulsion obtained from step (iii) above which is to be administered to the patient as a drink and that said administration should take place upon formation of the emulsion or shortly thereafter in order to avoid coalescence, creaming or phase separation. The self-dispersing drug delivery system comprising an active compound is thus prepared for administration as an emulsion after addition to an aqueous solution.

According to the present invention, the mixture obtained from step (ii) preferably is at least partially an emulsion.

According to the present invention, the active compound may be sensitive to hydrolysis. Preferably, the active compound is a lipophilic compound. As discussed herein, lipophilic compounds comprise those compounds which are soluble in polar lipids. In a more preferred embodiment, the active compound additionally is sensitive to hydrolysis.

The present invention is particularly advantageous for lipophilic compounds which are sensitive to hydrolysis.

Therefore, the present invention provides a process for dosing a self-dispersing drug delivery system comprising an active compound as described above, wherein the active compound is a compound which is sensitive to hydrolysis. Furthermore, the present invention provides a process for dosing a self-dispersing drug delivery system comprising an active compound as described above, wherein the active compound is a lipophilic compound which is sensitive to hydrolysis.

According to the present invention, the active compound can include any physiologically or pharmacologically active substance that produces a localized or systemic effect in a patient. The active compound can be selected from antibiotics, antiviral agents, anepileptics, analgesics, anti-inftammatory agents and bronchodilators, and may be inorganic and organic compounds, including, without limitation, drugs which act on the peripheral nerves, adrenergic receptors, cholinergic receptors, the skeletal muscles, the cardiovascular system, smooth muscles, the blood circulatory system, synoptic sites, neuroeffector junctional sites, endocrine and hormone systems, the immunological system, the reproductive system, the skeletal system, autacoid systems, the alimentary and excretory systems, the histamine system and the central nervous system. Suitable active compounds may be selected from, for example, polysaccharides, steroids, hypnotics and sedatives, psychic energizers, tranquilizers, anticonvulsants, muscle relaxants, antiparkinson agents, analgesics, anti-inflammatories, muscle contractants, antimicrobials, antimalarials, hormonal agents including contraceptives, sympathomimetics, polypeptides and proteins capable of eliciting physiological effects, diuretics, lipid regulating agents, antiandrogenic agents, antiparasitics, neoplastics, antineoplastics, hypoglycemics, nutritional agents and supplements, growth supplements, fats, ophthalmics, antienteritis agents, electrolytes and diagnostic agents.

Examples of active compounds according to the present invention include prochlorperazine edisylate, ferrous sulfate, aminocaproic acid, mecamylamine hydrochloride, procainamide hydrochloride, amphetamine sulfate, methamphetamine hydrochloride, benzphetamine hydrochloride, isoproterenol sulfate, phenmetrazine hydrochloride, bethanechol chloride, methacholine chloride, pilocarpine hydrochloride, atropine sulfate, scopolamine bromide, isopropamide iodide, tridihexethyl chloride, phenformin hydrochloride, methylphenidate hydrochloride, theophylline cholinate, cephalexin hydrochloride, diphenidol, meclizine hydrochloride, prochlorperazine maleate, phenoxybenzamine, thiethylperazine maleate, anisindione, diphenadione erythrityl tetranitrate, digoxin, isoflurophate, acetazolamide, methazolamide, bendroflumethiazide, chlorpropamide, tolazamide, chlormadinone acetate, phenaglycodol, allopurinol, aluminum aspirin, methotrexate, acetyl sulfisoxazole, hydrocortisone, hydrocorticosterone acetate, cortisone acetate, dexamethasone and its derivatives such as betamethasone, triamcinolone, methyltestosterone, 17b-estradiol, ethinyl estradiol, ethinyl estradiol 3-methyl ether, prednisolone, 17-b-hydroxyprogesterone acetate, 19-nor-progesterone, norgestrel, norethindrone, norethisterone, norethiederone, progesterone, norgesterone, norethynodrel, aspirin, acetaminophen, indomethacin, naproxen, fenoprofen, sulindac, indoprofen, nitroglycerin, isosorbide dinitrate, propranolol, timolol, atenolol, alprenolol, cimetidine, clonidine, imipramine, levodopa, chlorpromazine, methyldopa, dihydroxyphenylalanine, calcium gluconate, ketoprofen, ibuprofen, cephalexin, erythromycin, haloperidol, zomepirac, ferrous lactate, vincamine, phenoxybenzamine, diltiazem, milrinone, captropril, mandol, quanbenz, hydrochlorothiazide, ranitidine, flurbiprofen, fenbufen, fluprofen, tolmetin, alclofenac, mefenamic, flufenamic, difuninal, nimodipine, nitrendipine, nisoldipine, nicardipine, felodipine, lidoflazine, tiapamil, gallopamil, amlodipine, mioflazine, lisinopril, enalapril, captopril, ramipril, enalaprilat, famotidine, nizatidine, sucralfate, etintidine, tetratolol, minoxidil, chlordiazepoxide, diazepam, amitriptyline, and imipramine. Further examples of the substance comprise proteins and peptides which include, but are not limited to, insulin, colchicine, glucagon, thyroid stimulating hormone, parathyroid and pituitary hormones, calcitonin, renin, prolactin, corticotrophin, thyrotropic hormone, follicle stimulating hormone, chorionic gonadotropin, gonadotropin releasing hormone, bovine somatotropin, porcine somatropin, oxytocin, vasopressin, prolactin, somatostatin, lypressin, pancreozymin and luteinizing hormone.

It is to be understood that more than one active compound may be incorporated into the self-dispersing drug delivery systems according to the invention.

The active compound can be in various forms, such as soluble and insoluble charged or uncharged molecules, components of molecular complexes or nonirritating, pharmacologically acceptable salts.

The amount of active compound contained in the compositions according to the present invention will be that amount necessary to deliver a therapeutically effective amount of the active compound to achieve the desired result.

According to the present invention, the self-dispersing drug delivery system can also comprise further compounds which enhance for example the activity, taste or appearance of the emulsions obtained. According to the present invention, it is also possible that the aqueous solution comprises further compounds which improve any of the properties of the emulsions obtained. It is also possible that both, the self-dispersing drug delivery system and the aqueous solution comprise further compounds.

The self-dispersing drug delivery system may for example be associated with any pharmaceutical excipient to form a dosage form, which can be administered to animals or humans.

In the context of the present invention, the self-dispersing drug delivery system and/or the aqueous solution can comprise any suitable further compound, for example pharmaceutical excipients, diluents, sweeteners, flavouring agents, and colouring agents.

Therefore, the present invention provides a process for dosing a self-dispersing drug delivery system comprising an active compound as described above, wherein the self-dispersing drug delivery system and/or the aqueous solution contains at least one compound selected from the group consisting of pharmaceutical excipients, diluents, sweeteners, flavouring agents, and colouring agents.

The dosage dispenser according to the present invention is suitable for dosing a defined amount. According to the present invention, the defined amount can be dosed in a single stroke. In this case, the dosage dispenser suitably has means to dose a defined amount in a single stroke.

In particular, the dosage dispenser preferably administers uniform dosage units with a content uniformity of F between 0.900 and 1.100, preferably between 0.970 and 1.030 as discussed above.

According to the present invention, it is also possible that the dosage dispenser has optical and/or tactile marks, preferably on the container, to determine and preferably to adjust the amount dosed in one stroke.

Therefore, the present invention provides a process for dosing a self-dispersing drug delivery system comprising an active compound as described above, wherein the defined amount of the self-dispersing drug delivery system in step (i) is obtained in a single-stroke of the dosage dispenser.

The present invention also provides a process for dosing a self-dispersing drug delivery system comprising an active compound as described above, wherein the dosage dispenser has marks to determine the amount of self-dispersing drug delivery system dosed.

According to another aspect, the present invention provides a process for preparing a pharmaceutical composition. In particular, the present invention provides a process for preparing a pharmaceutical composition comprising the steps of
(i) using a dosage dispenser to dose a defined amount of a self-dispersing drug delivery system comprising an active compound;
(ii) adding the defined amount of the self-dispersing drug delivery system comprising an active compound obtained in step (i) to an aqueous solution to obtain an emulsion.

The emulsion obtained according to step (ii) can be used as pharmaceutical composition without further treatment according to the present invention. The self-dispersing drug delivery system comprising an active compound is thus prepared for administration as an emulsion after addition to an aqueous solution.

With regard to preferred embodiments, reference is made to the above disclosure.

The present invention also provides a process for preparing a pharmaceutical composition as described above, wherein the self-dispersing drug delivery system is a self-emulsifying drug delivery system. Therefore, the present invention also provides a process for preparing a pharmaceutical composition comprising the steps of
(i) using a dosage dispenser to dose a defined amount of a self-emulsifying drug delivery system comprising an active compound;
(ii) adding the defined amount of the self-emulsifying drug delivery system comprising an active compound obtained in step (i) to an aqueous solution to obtain an emulsion.

Furthermore, the present invention also provides a process for preparing a pharmaceutical composition as described above, wherein the process additionally comprises the step
(iii) agitating the mixture obtained from step (ii) comprising the aqueous solution and the self-dispersing drug delivery system comprising an active compound.

The skilled person will understand that it is in each case the emulsion obtained which is to be administered to the patient as a drink and that said administration should take place upon formation of the emulsion or shortly thereafter in order to avoid coalescence, creaming or phase separation.

The present invention also provides a process for preparing a pharmaceutical composition as described above, wherein the active compound is a compound which is sensitive to hydrolysis. Furthermore, the present invention provides a process for preparing a pharmaceutical composition as described above, wherein the active compound is a lipophilic compound which is sensitive to hydrolysis.

With regard to the active compound, reference is made to the above disclosure of suitable compounds in the context of the present invention.

The present invention also provides a process for preparing a pharmaceutical composition as described above, wherein the self-dispersing drug delivery system and/or the aqueous solution contains at least one compound selected from the group consisting of pharmaceutical excipients, diluents, sweeteners, flavouring agents, and colouring agents.

According to another aspect, the present invention also provides the use of a dosage dispenser for dosing a self-dispersing drug delivery system comprising an active compound.

Again, reference is made to the above disclosure with respect to preferred embodiments.

The present invention also provides the use of a dosage dispenser for dosing a self-dispersing drug delivery system comprising an active compound as disclosed above, wherein the self-dispersing drug delivery system is a self-emulsifying drug delivery system. In particular, the present invention also provides the use of a dosage dispenser for dosing a self-emulsifying drug delivery system comprising an active compound.

An advantage of the present invention is the shelf-stability of the self-dispersing drug delivery systems, which preferably includes both chemical and physical stability. Chemical stability includes stability of the composition against chemical degradation, e.g., hydrolysis, oxidation/reduction, photolysis, etc. Physical stability refers to stability against change in physical form, including, e.g., no, or essentially no, crystallization of the active ingredient from the solution. Self-dispersing drug delivery systems are preferably shelf-stable for at least the interval from when the composition is manufactured to the time when the patient could reasonably be expected to take the composition. Compositions are also preferably shelf-stable when subjected to typical manufacturing and marketing conditions, such as storage and transportation. Compositions of the present invention are preferably shelf-stable for at least three months under accelerated challenge conditions (40° C and 75% relative humidity) and for at least two years under recommended storage conditions, such as ambient temperature.

Therefore, it is possible to store the self-dispersing drug delivery system comprising the active compound in the dosage dispenser according to the present invention and provide the defined amount of the self-dispersing drug delivery system comprising the active compound when needed.

The present invention thus provides the use of a dosage dispenser for dosing a self-dispersing drug delivery system comprising an active compound as disclosed above, wherein the active compound is a compound which is sensitive to hydrolysis. Furthermore, the present invention provides the use of a dosage dispenser for dosing a self-dispersing drug delivery system comprising an active compound as disclosed above, wherein the active compound is a lipophilic compound which is sensitive to hydrolysis.

Preferred active compounds in the context of the present invention are disclosed above.

Additionally, the present invention provides the use of a dosage dispenser for dosing a self-dispersing drug delivery system comprising an active compound as disclosed above, wherein the dosage dispenser has marks to determine the amount of self-dispersing drug delivery system dosed.

According to another aspect, the present invention also provides a dosage dispenser containing a self-dispersing drug delivery system comprising an active compound. In particular, the present invention provides a dosage dispenser containing a self-emulsifying drug delivery system comprising an active compound. The self-dispersing drug delivery system comprising an active compound is prepared for administration as an emulsion after addition to an aqueous solution.

Therefore, the present invention also provides the dosage dispenser containing a self-dispersing drug delivery system as disclosed above, wherein the active compound is a compound which is sensitive to hydrolysis, in particular a lipophilic compound which is sensitive to hydrolysis.

Preferred active compounds are mentioned above.

Furthermore, the present invention also provides the dosage dispenser containing a self-dispersing drug delivery system as disclosed above, wherein the dosage dispenser has marks to determine the amount of self-dispersing drug delivery system dosed.

Preferably, the dosage dispenser according to the present invention is in a form which makes it usable for dosing the self-dispersing drug delivery system to form the emulsion which in turn is applied as the pharmaceutical composition. Therefore, the present invention also provides the dosage dispenser containing a self-dispersing drug delivery system as disclosed above, wherein the dosage dispenser is used for dosing a self-dispersing drug delivery system comprising an active compound.

According to a further aspect, the present invention also provides a combination comprising
(a) a dosage dispenser containing a self-dispersing drug delivery system comprising an active compound and being capable of dosing a defined amount of a self-dispersing drug delivery system comprising an active compound, and
(b) a product leaflet containing instructions that a defined amount of said shelf-dispersing drug delivery system is to be dispensed into an aqueous solution to obtain an emulsion and that said obtained emulsion is to be administered as a drink to the patient.

According to another aspect, the present invention also provides a combination comprising
(A) a dosage dispenser containing a self-dispersing drug delivery system comprising an active compound and being capable of dosing a defined amount of a self-dispersing drug delivery system comprising an active compound, and
(B) a container with a potable amount of an aqueous solution, wherein the defined amount of said self-dispersing drug delivery system is to be dispensed to form an emulsion which can be administered as a drink.

The combinations according to the present invention have the advantage that they allow for adjusting the dosage of a self-dispersing drug delivery system comprising an active compound to the particular needs of a patient combined with storage stability of the self-dispersing drug delivery system comprising an active compound.

In the context of the present invention, it is also possible that the product leaflet is combined with the packaging or the dosage dispenser itself.

The use of a container with a potable amount of an aqueous solution according to the present invention has the advantage that no further instruments or containers are needed to apply the self-dispersing drug delivery system comprising the active compound. According to another embodiment of the present invention the dosage dispenser containing the self-dispersing drug delivery system comprising an active compound is combined with a container suitable for measuring a potable amount of an aqueous solution. This container is preferably suitable for multiple use.

Therefore, according to a further embodiment, the present invention also provides a combination comprising
(A) a dosage dispenser containing a self-dispersing drug delivery system comprising an active compound and being capable of dosing a defined amount of a self-dispersing drug delivery system comprising an active compound, and
(B') a container suitable for measuring a potable amount of an aqueous solution, wherein the defined amount of said self-dispersing drug delivery system is to be dispensed in the potable amount of an aqueous solution to form an emulsion which can be administered as a drink.

Preferably, the potable amount of an aqueous solution is adjusted to the amount of the self-dispersing drug delivery system which is to be dispensed in said potable amount of the aqueous solution. The self-dispersing drug delivery system comprising an active compound is thus prepared for administration as an emulsion after addition to an aqueous solution.

According to the present invention, it is also possible to combine the dosage dispenser with a leaflet and a container as disclosed above. Therefore, the present invention also provides a combination comprising
(a) a dosage dispenser containing a self-dispersing drug delivery system comprising an active compound and being capable of dosing a defined amount of a self-dispersing drug delivery system comprising an active compound,
(b) a product leaflet containing instructions that a defined amount of said self-dispersing drug delivery system is to be dispensed into an aqueous solution to obtain an emulsion and that said obtained emulsion is to be administered as a drink to the patient, and
(c) a container with a potable amount of an aqueous solution, wherein the defined amount of said self-dispersing drug delivery system is to be dispensed to form an emulsion which can be administered as a drink.

Preferred active compounds in the context of the present invention are disclosed above. With respect to preferred embodiments, reference is made to the above disclosure.

Preferably, the aqueous solution is water or a juice. Therefore, according to a preferred embodiment, the present invention is directed to a combination as disclosed above, wherein the aqueous solution is selected from the group consisting of juices, in particular orange or apple juice.

The viscosity of the self-dispersing drug delivery system comprising an active compound can vary in wide ranges depending on the dosage dispenser used. Preferably it is in the range of 500 mPa^{·}s to 80 000 mPa^{·}s. Therefore, according to a preferred embodiment, the present invention is directed to a combination as disclosed above, wherein the viscosity of the self-dispersing drug delivery system comprising an active compound is in the range of 500 mPa^{·}s to 80 000 mPa^{·}s.

According to a further aspect, the present invention also provides a method of treating a disease condition comprising administering to a patient in need of such treating a therapeutically effective amount of the pharmaceutical composition obtainable according to a process according to the present invention.

In another aspect, the pharmaceutical composition may be administered as a prophylactic measure, rather than a therapeutic measure.

A "therapeutically effective amount" is considered to be that amount which effects a reduction in one or more symptom or effect associated with the disease condition. A "prophylactically effective amount" is considered to be that amount that improves or prevents a change or worsening in a parameter useful in the prediction of the development of the disease condition. The determination of therapeutically or prophylactically effective amounts of the pharmaceutical composition of the invention is accomplished through conventional techniques. Factors to be considered in determining the appropriate dose for each patient include, but are not limited to the patient's age, weight, and gender; the gravity of the patient's condition; the route of administration; the elements of the pharmaceutical composition, particularly the identity of the active compound.

According to the present invention, each dosage form may include, apart from the essential components of the composition conventional pharmaceutical excipients, diluents, sweeteners, flavouring agents, colouring agents and any other inert ingredients regularly included in dosage forms intended for oral administration.

Compositions of the present invention are preferably administered to mammals, such as dog, cat, horse, pig, mice, rat and especially humans. It is preferred that the pharmaceutical compositions of the present invention are administered orally.

The present invention also provides a pharmaceutical formulation comprising the pharmaceutical composition obtainable according to a process according to the present invention and at least one pharmaceutically acceptable excipient.

Therefore, the present invention also provides a formulation as described above for treating a disease condition in a mammal, in particular in man.

The present invention further provides the use of a therapeutically effective amount of the pharmaceutical composition obtainable according to a process according to the present invention and at least one pharmaceutically acceptable excipient for the preparation of a medicament for treating a disease condition, preferably wherein said medicament is to be administered to a patient in need thereof

The following examples illustrate the process of the present invention and are not intended to limit the scope of the invention set forth in the claims appended thereto.

### Examples

### Example 1:

0.200 g azithromycin dihdrate, 1.800 g glycerin (water free, Ph. Eur.), 0.050 g Strawberry 501094A (liquid, from company Firmenich), and 0.008 g sodium cyclamate were mixed and stirred for about 15 minutes. The resulting mixture was degassed under reduced pressure.

The resulting mixture could be dosed using a dosage dispenser.

### Example 2:

0.012 g lecithin (EPIKURON 100) and 0.050 g Strawberry 501094A (liquid, from company Firmenich) were dissolved in 1.500 g Miglyol 810 giving an oily solution. 0.200 g azithromycin dihdrate and 1.500 g saccharose (powdered, Ph. Eur) were milled together with the oily solution in a ball mill.

The resulting mixture could be dosed using a dosage dispenser.

### Example 3:

0.001 g sodium saccharin (powdered, EP) were dissolved in 0.200 g glycerin (water free, Ph. Eur.) at elevated temperature of about 60°C to 70°C. The resulting solution was cooled to room temperature. 1.150 g Labrafil M 1944, 0.350 g Plurol Oleique, 0.010 g Tween 80, and 0.020 g ethyl oleate were mixed and added to the solution. The resulting mixture was milled together with 0.200 g amoxicillin trihydrate (powdered) in a ball mill.

The resulting mixture could be dosed using a dosage dispenser.

## Claims

1. A process for dosing a liquid self-dispersing drug delivery system comprising an active compound, said process comprising the step of
(i) using a dosage dispenser comprising a container and a pumping device to dose a defined amount of said liquid self-dispersing drug delivery system comprising an active compound, and
(ii) adding the defined amount of the self-dispersing drug delivery system comprising an active compound obtained in step (i) to an aqueous solution before administration to obtain an emulsion suitable for administration to a patient as a drink.

2. The process according to claim 1, wherein the process additionally comprises the step
(iii) agitating the mixture obtained from step (ii) comprising the aqueous solution and the self-dispersing drug delivery system comprising an active compound.

3. The process according claim 1 or 2, wherein the liquid self-dispersing drug delivery system is a self-emulsifying drug delivery system.

4. The process according to any of claims I to 3, wherein the active compound is a compound which is sensitive to hydrolysis.

5. The process according to any of claims 1 to 4, wherein the active compound is a lipophilic compound which is sensitive to hydrolysis.

6. The process according to any of claims 1 to 5, wherein the liquid self-dispersing drug delivery system and/or the aqueous solution contains at least one compound selected from the group consisting of pharmaceutical excipients, diluents, sweeteners, flavouring agents, and colouring agents.

7. The process according to any of claims 1 to 6, wherein the defined amount of the liquid self-dispersing drug delivery system in step (i) is obtained in a single-stroke of the dosage dispenser.

8. The process according to any of claims 1 to 7, wherein the dosage dispenser has marks to determine the amount of self-dispersing drug delivery system dosed.

9. The process according to any of claims 1 to 8, wherein step (ii) is carried out shortly before administration, such that coalescence, creaming or phase separation is avoided.

10. A process for preparing a pharmaceutical composition comprising the steps of
(i) using a dosage dispenser comprising a container and a pumping device to dose a defined amount of a liquid self-dispersing drug delivery system comprising an active compound;
(ii) adding the defined amount of the liquid, self-dispersing drug delivery system comprising an active compound obtained in step (i) to an aqueous solution before administration to obtain a pharmaceutical composition in form of an emulsion suitable for administration to a patient as a drink.

11. The process according to claim 10, wherein the process additionally comprises the step
(iii) agitating the mixture obtained from step (ii) comprising the aqueous solution and the self-dispersing drug delivery system comprising an active compound.

12. The process according to any of claims 10 or 11, wherein the liquid self-dispersing drug delivery system is a self-emulsifying drug delivery system.

13. The process according to any of claims 10 to 12, wherein the active compound is a compound which is sensitive to hydrolysis.

14. The process according to any of claims 10 to 13, wherein the active compound is a lipophilic compound which is sensitive to hydrolysis.

15. The process according to any of claims 10 to 14, wherein the liquid self-dispersing drug delivery system and/or the aqueous solution contains at least one compound selected from the group consisting of pharmaceutical excipients, diluents, sweeteners, flavouring agents, and colouring agents.

16. The process according to any of claims 10 to 15, wherein step (ii) is carried out shortly before administration, such that coalescence, creaming or phase separation is avoided.

17. Use of a dosage dispenser comprising a container and a pumping device for dosing a liquid self-dispersing drug delivery system comprising an active compound for preparing a pharmaceutical composition in form of an emulsion, suitable for administration to a patient as a drink.

18. The use according to claim 17, wherein the liquid self-dispersing drug delivery system is a self-emulsifying drug delivery system.

19. The use according to claim 17 or 18, wherein the active compound is a compound which is sensitive to hydrolysis.

20. The use according to any of claims 17 to 19, wherein the dosage dispenser has marks to determine the amount of self-dispersing drug delivery system dosed.

21. The use according to any of claims 17 to 20, wherein the pharmaceutical composition is prepared shortly before administration, such that coalescence, creaming or phase separation is avoided.

## Patentansprüche

1. Verfahren zum Dosieren eines flüssigen selbst-dispergierenden Arzneistoffzuführungssystems, das einen Wirkstoff umfasst, wobei das Verfahren die Schritte umfasst
(i) Verwenden eines Dosierungsspenders, der einen Behälter und eine Pumpvorrichtung umfasst, zum Dosieren einer definierten Menge des flüssigen selbst-dispergierenden Arzneistoffzuführungssystems, das einen Wirkstoff umfasst, und
(ii) Zugeben der im Schritt (i) erhaltenen definierten Menge des selbst-dispergierenden Arzneistoffzuführungssystems, das einen Wirkstoff umfasst, zu einer wässrigen Lösung vor der Verabreichung, um eine Emulsion zu erhalten, die für die Verabreichung an einen Patienten als Getränk geeignet ist.

2. Verfahren nach Anspruch 1, wobei das Verfahren zusätzlich den Schritt umfasst
(iii) Rühren des aus Schritt (ii) erhaltenen Gemischs, das die wässrige Lösung und das selbst-dispergierende Arzneistoffzuführungssystem, das einen Wirkstoff umfasst, umfasst.

3. Verfahren nach Anspruch 1 oder 2, wobei das flüssige selbst-dispergierende Arzneistoffzuführungssystem ein selbst-emulgierendes Arzneistoffzuführungssystem ist.

4. Verfahren nach einem der Ansprüche 1 bis 3, wobei der Wirkstoff eine hydrolyseempfindliche Verbindung ist.

5. Verfahren nach einem der Ansprüche 1 bis 4, wobei der Wirkstoff eine lipophile Verbindung ist, die hydrolyseempfindlich ist.

6. Verfahren nach einem der Ansprüche 1 bis 5, wobei das flüssige selbst-dispergierende Arzneistoffzuführungssystem und/oder die wässrige Lösung mindestens eine Verbindung enthält, die aus der Gruppe ausgewählt ist, bestehend aus pharmazeutischen Hilfsstoffen, Verdünnungsmitteln, Süßstoffen, Geschmacksstoffen und Farbstoffen.

7. Verfahren nach einem der Ansprüche 1 bis 6, wobei die definierte Menge des flüssigen selbst-dispergierenden Arzneistoffzuführungssystems im Schritt (i) bei einer einzigen Betätigung des Dosierungsspenders erhalten wird.

8. Verfahren nach einem der Ansprüche 1 bis 7, wobei der Dosierungsspender Markierungen zur Bestimmung der Menge an dosiertem selbst-dispergierendem Arzneistoffzuführungssystem besitzt.

9. Verfahren nach einem der Ansprüche 1 bis 8, wobei Schritt (ii) kurz vor der Verabreichung durchgeführt wird, so dass Koaleszenz, Aufrahmen oder Phasenentmischung verhindert werden.

10. Verfahren zur Herstellung einer pharmazeutischen Zusammensetzung, das die Schritte umfasst
(i) Verwenden eines Dosierungsspenders, der einen Behälter und eine Pumpvorrichtung umfasst, zum Dosieren einer definierten Menge eines flüssigen selbst-dispergierenden Arzneistoffzuführungssystems, das einen Wirkstoff umfasst,
(ii) Zugeben der im Schritt (i) erhaltenen definierten Menge des flüssigen selbst-dispergierenden Arzneistoffzuführungssystems, das einen Wirkstoff umfasst, zu einer wässrigen Lösung vor der Verabreichung, unter Erhalt einer pharmazeutischen Zusammensetzung in Form einer Emulsion, die für die Verabreichung an einen Patienten als Getränk geeignet ist.

11. Verfahren nach Anspruch 10, wobei das Verfahren zusätzlich den Schritt umfasst
(iii) Rühren des aus Schritt (ii) erhaltenen Gemischs, das die wässrige Lösung und das selbst-dispergierende Arzneistoffzuführungssystem, das einen Wirkstoff umfasst, umfasst.

12. Verfahren nach einem der Ansprüche 10 oder 11, wobei das flüssige selbst-dispergierende Arzneistoffzuführungssystem ein selbst-emulgierendes Arzneistoffzuführungssystem ist.

13. Verfahren nach einem der Ansprüche 10 bis 12, wobei der Wirkstoff eine hydrolyseempfindliche Verbindung ist.

14. Verfahren nach einem der Ansprüche 10 bis 13, wobei der Wirkstoff eine lipophile Verbindung ist, die hydrolyseempfindlich ist.

15. Verfahren nach einem der Ansprüche 10 bis 14, wobei das flüssige selbst-dispergierende Arzneistoffzuführungssystem und/oder die wässrige Lösung mindestens eine Verbindung enthält, die aus der Gruppe ausgewählt ist, bestehend aus pharmazeutischen Hilfsstoffen, Verdünnungsmitteln, Süßstoffen, Geschmacksstoffen und Farbstoffen.

16. Verfahren nach einem der Ansprüche 10 bis 15, wobei Schritt (ii) kurz vor der Verabreichung durchgeführt wird, so dass Koaleszenz, Aufrahmen oder Phasenentmischung verhindert werden.

17. Verwendung eines Dosierungsspenders, der einen Behälter und eine Pumpvorrichtung umfasst, zum Dosieren eines flüssigen selbst-dispergierenden Arzneistoffzuführungssystems, das einen Wirkstoff umfasst, zur Herstellung einer pharmazeutischen Zusammensetzung in Form einer Emulsion, die für die Verabreichung an einen Patienten als Getränk geeignet ist.

18. Verwendung nach Anspruch 17, wobei das flüssige selbst-dispergierende Arzneistoffzuführungssystem ein selbst-emulgierendes Arzneistoffzuführungssystem ist.

19. Verwendung nach Anspruch 17 oder 18, wobei der Wirkstoff eine hydrolyseempfindliche Verbindung ist.

20. Verwendung nach einem der Ansprüche 17 bis 19, wobei der Dosierungsspender Markierungen zur Bestimmung der Menge an dosiertem selbst-dispergierendem Arzneistoffzuführungssystem besitzt.

21. Verwendung nach einem der Ansprüche 17 bis 20, wobei die pharmazeutische Zusammensetzung kurz vor der Verabreichung hergestellt wird, so dass Koaleszenz, Aufrahmen oder Phasenentmischung verhindert werden.

## Revendications

1. Procédé pour le dosage d'un système d'administration de médicament auto-dispersant liquide comprenant un composé actif, ledit procédé comprenant l'étape consistant à
(i) utiliser un distributeur de doses comprenant un récipient et un dispositif de pompage pour doser une quantité définie dudit système d'administration de médicament auto-dispersant liquide comprenant un composé actif et
(ii) ajouter la quantité définie du système d'administration de médicament auto-dispersant comprenant un composé actif obtenue, dans l'étape (i) à une solution aqueuse avant l'administration pour obtenir une émulsion appropriée pour l'administration à un patient en tant que boisson.

2. Procédé selon la revendication 1, le procédé comprenant de plus l'étape consistant à
(iii) agiter le mélange obtenu à partir de l'étape (ii) comprenant la solution aqueuse et le système d'administration de médicament auto-dispersant comprenant un composé actif.

3. Procédé selon la revendication 1 ou 2, dans lequel le système d'administration de médicament auto-dispersant liquide est un système d'administration de médicament auto-émulsifiant.

4. Procédé selon l'une quelconque des revendications 1 à 3, dans lequel le composé actif est un composé qui est sensible à l'hydrolyse.

5. Procédé selon l'une quelconque des revendications 1 à 4, dans lequel le composé actif est un composé lipophile qui est sensible à l'hydrolyse.

6. Procédé selon l'une quelconque des revendications 1 à 5, dans lequel le système d'administration de médicament auto-dispersant liquide et/ou la solution aqueuse contient au moins un composé choisi dans le groupe constitué par les excipients pharmaceutiques, les diluants, les édulcorants, les agents aromatisants et les agents colorants.

7. Procédé selon l'une quelconque des revendications 1 à 6, dans lequel la quantité définie du système d'administration de médicament auto-dispersant liquide dans l'étape (i) est obtenue en un seul actionnement du distributeur de doses.

8. Procédé selon l'une quelconque des revendications 1 à 7, dans lequel le distributeur de doses a des marques pour déterminer la quantité de système d'administration de médicament auto-dispersant dosé.

9. Procédé selon l'une quelconque des revendications 1 à 8, dans lequel l'étape (ii) est effectuée peu de temps avant l'administration, de sorte qu'une coalescence, un crémage ou une séparation de phases est évité.

10. Procédé pour la préparation d'une composition pharmaceutique comprenant les étapes consistant à
(i) utiliser un distributeur de doses comprenant un récipient et un dispositif de pompage pour doser une quantité définie d'un système d'administration de médicament auto-dispersant liquide comprenant un composé actif ;
(ii) ajouter la quantité définie du système d'administration de médicament auto-dispersant liquide comprenant un composé actif obtenue dans l'étape (i) à une solution aqueuse avant l'administration pour obtenir une composition pharmaceutique sous forme d'une émulsion appropriée pour l'administration à un patient en tant que boisson.

11. Procédé selon la revendication 10, le procédé comprenant de plus l'étape consistant à
(iii) agiter le mélange obtenu à partir de l'étape (ii) comprenant la solution aqueuse et le système d'administration de médicament auto-dispersant comprenant un composé actif.

12. Procédé selon l'une quelconque des revendications 10 ou 11, dans lequel le système d'administration de médicament auto-dispersant liquide est un système d'administration de médicament auto-émulsifiant.

13. Procédé selon l'une quelconque des revendications 10 à 12, dans lequel le composé actif est un composé qui est sensible à l'hydrolyse.

14. Procédé selon l'une quelconque des revendications 10 à 13, dans lequel le composé actif est un composé lipophile qui est sensible à l'hydrolyse.

15. Procédé selon l'une quelconque des revendications 10 à 14, dans lequel le système d'administration de médicament auto-dispersant liquide et/ou la solution aqueuse contient au moins un composé choisi dans le groupe constitué par les excipients pharmaceutiques, les diluants, les édulcorants, les agents aromatisants et les agents colorants.

16. Procédé selon l'une quelconque des revendications 10 à 15, dans lequel l'étape (ii) est effectuée peu de temps avant l'administration, de sorte qu'une coalescence, un crémage ou une séparation de phases est évité.

17. Utilisation d'un distributeur de doses comprenant un récipient et un dispositif de pompage pour le dosage d'un système d'administration de médicament auto-dispersant liquide comprenant un composé actif pour la préparation d'une composition pharmaceutique sous forme d'une émulsion, appropriée pour l'administration à un patient en tant que boisson.

18. Utilisation selon la revendication 17, dans laquelle le système d'administration de médicament auto-dispersant liquide est un système d'administration de médicament auto-émulsifiant.

19. Utilisation selon la revendication 17 ou 18, dans laquelle le composé actif est un composé qui est sensible à l'hydrolyse.

20. Utilisation selon l'une quelconque des revendications 17 à 19, dans laquelle le distributeur de doses a des marques pour déterminer la quantité de système d'administration de médicament auto-dispersant dosé.

21. Utilisation selon l'une quelconque des revendications 17 à 20, dans laquelle la composition pharmaceutique est préparée peu de temps avant l'administration, de sorte qu'une coalescence, un crémage ou une séparation de phases est évité.
